# EUROPEAN PATENT SPECIFICATION

(11) **EP 3 501 489 B1**
(45) Date of publication and mention of the grant of the patent: **01.11.2023**
(21) Application number: 17842531.0
(22) Date of filing: 13.02.2017
(51) Int. Cl.: A61K 8/40, A61K 8/362, A61K 8/34, A61Q 5/00, A61K 8/41, A61Q 5/12

(54) **METHOD FOR STRENGTHENING AND REPAIRING HAIR, AND KIT THEREFOR**
VERFAHREN ZUR STÄRKUNG UND REPARATUR VON HAAREN UND KIT DAFÜR
PROCÉDÉ DE RENFORCEMENT ET DE RÉPARATION DE CHEVEUX, ET KIT ASSOCIÉ

(30) Priority: 22.08.2016 CN 201610701375
(43) Date of publication of application: 26.06.2019
(73) Proprietor: LIW Patent Company limited by guarantee, Dublin 3 (IE)
(72) Inventor: HOFFMANN, Katherina, Sydney, NSW 2747 (AU)
(74) Representative: Meyer-Dulheuer MD Legal Patentanwälte PartG mbB
(86) International application number: PCT/CN2017/073367
(87) International publication number: WO 2018/036105

(56) References cited:
- CN-A- 105 267 066
- CN-A- 105 267 066
- CN-A- 105 496 804
- CN-A- 105 496 804
- CN-A- 106 265 109

## Description

### FIELD OF THE INVENTION

A method, composition and kit for strengthening hair including, while at the same time providing excellent hair feel characteristics, providing a composition capable of crosslinking the amino-acid groups of keratin fibers, comprising also the mixing of such crosslinking composition with commercially available hair color or hair bleaching formulations.

### BACKGROUND OF THE INVENTION

The invention is related to hair treatment agents, which have at least bi-functional active ingredients, wherein the active ingredients react with the amino-acid groups of the hair and thereby improve the quality of the hair in various ways without compromising hair feel. The current invention is also related to a method for improving the condition of the hair.

Constant bleach, permanent waves and coloration, and sometimes even frequent hair wash with degreasing surfactants or other active ingredients can result in damages of the hair structure. The hair turns brittle and loses its luster. In addition, combing the hair charges the hair electrostatically and the roughened surface of the hair leads to entanglement of the hairs. Combing will thus become more difficult.

Hair treatment agents with an nourishing effect of the hair and which can facilitate hair combing have therefore become important and were known in the European patent application EP 234261. Such agents are, for instance, in the form of clear hair care rinsing solution or in the form of so called "cream-rinses" emulsion distributed on the still wet hair after hair wash, left on the hair for a couple of minutes to an hour and the hair are then rinsed with water.

Hair treatment agents on the basis of the above mentioned conditioning active ingredients, however, only show satisfactory results when treating dry and spongy hair. For the treatment of easily greasy hair, they tend to be less satisfactory, since their application makes the hair greasier so that the durability of the hair style decreases. It is also known that the durability of the hair style is dependent on the disulfide bonds, which are unstable and can be easily reduced to sulfhydryl groups under reducing condition. There have been plenty attempts to reestablish the disulfide bonds through the introduction of oxidizing agents. A lot of agents which improve the stability of the permanent waves are based on such technology. Also a great number of patents and patent applications are directed to reestablishment of disulfide bonds, such as the US patent 9,095,518 B2, the US patent applications 2015034119 A1, 201537270 A1, 201537271 A1 und WO 2015017768 A1. Nevertheless, the hair treatment agents which are described in these documents have lots of room for improvements.

It has also been attempted to apply amino acids, such as a weak acid mixture of different amino acids and vitamins (US-PS 4201235), as a hair conditioning component to avoid the disadvantages of the known hair treatment agents. However, the production of such a mixture of different vitamins and amino acids is troublesome and expensive.

Furthermore, the application of keratin-hydrolyzate and citric acid in a "neutralizing shampoo" is known from the literature "Cosmetics and Toileteries" Vol 98 (1983), S. 59-68. This shampoo, however, possesses only a low hair care effect and leads to a strong dehydration of the hair. For this reason, it is necessary to apply once or repeatedly hair conditioning agents after hair wash.

It is also known from the literature W. Fassbender, Parfümerie & Kosmetik, 39 (1), S. 11-16 (1958) that the amino acid liquid, which contains 18 to 22 different amino acids, can be applied, for example, in weak acid adjusted hair treatment and hair care agent. The production of such amino acid liquid is carried out by fractionated hydrolysis of natural proteins and subsequent purification of the hydrolyzates obtained. It is therefore difficult to guarantee the constant composition of the liquid, which is important for the quality of the cosmetic agent.

It is also known from the Chinese patent application 201510645964.6 that at least bi-functional Bronsted acids and bases can be used in two distinctively different application steps and at two distinctively different pH values to ionically cross-link the hair from within. However, when the concentration ranges of said Chinese patent application 201510645964.6 are used, the hair feel is less than optimum.

The object of the invention is to provide a hair treatment agent and a hair treatment method which are based on bi-functional organic acids as well as bi-functional Bronsted-bases and which overcome the above described disadvantages. It is also an objective of the present invention to provide such hair treatment method in two distinctively different steps and at two distinctively different pH-values and in a concentration range that provides an optimum hair feel after the products are applied. It has been surprisingly found that the hair treatment agent containing the following combination meets these requirements:
a: a first formulation, comprising an at least bi-functional Bronsted-base of the general formulation X-R-Y, wherein X and Y are proton-acceptor groups and R is an organic spacer comprising 1 to 20 carbon atoms, and 0 to 5 oxygen atoms, and 0 to 5 nitrogen atoms, and X-R-Y having a molecular weight of less than 500 g/mol (1. Step)
b: a second formulation, comprising an at least bi-functional Bronsted-acid which can react with the amine groups of the hair (2. Step), and at least one fatty alcohol,
characterized in that the first formulation is applied before the second formulation is, and the first formulation has a pH of 7 to 12, and the at least bi-functional Bronsted-base of the first formulation is present in a concentration range of 0.1Gew.% to 25Gew.%, and the second formulation has a pH of 1.5 to 7, and the at least bi-functional Bronsted-acid of the second formulation is present in a concentration range of 0.01Gew.% to 0.99Gew.%, and the at least one fatty alcohol is present in a concentration range of 0.1Gew.% to 25Gew.%.

To adjust the pH values of the first and the second formulation, organic as well as inorganic acids and bases can be used. Those pH adjusting agents can be monofunctional or polyfunctional.

The proton-acceptor groups X and Y of the at least bi-functional Bronsted-base of step (a) are independently selected from the group of carboxylate, nitrate, hydrogen phosphate, phosphate, primary amine, secondary amine, tertiary amine, sulphate, and carbonate.

The at least bi-functional organic acid of step (b) is selected from the group of Oxalic-, Malonic-, Succinic-, Glutaric-, Adipic-, Pimeric-, Suberic-, Azelaic-, Sebacic-, Undecanedioic-, Dodecanedioic-, Methylmalonic-, Methylsuccinic-, 2-Methylglutaric-, Aspartic-, Maleic-, Fumaric-, Itaconic-, Mesaconic-, Methylmaleic-, Phthalic-, Isophthalic-, Terephthalic-, Malic-, Ketomalonic-, 4-Ketopimelic-, Citric-, Isocitric-, Actonitic-, Propane-1,2,3-tricarboxylic-, Trimesic-, Methanetetracarboxylic-, Ethylenetetracarboxylic-, Meso-butane-1,2,3,4-tetracarboxylic-, Furantetracarboxylic-acid, derivatives and mixtures thereof.

The at least one fatty alcohol is selected from the group of 1-Hexanol, 1-Heptanol, 1-Octanol, 1-Decanol, 1-Dodecanol, 1-Tetradecanol, 1-Hexadecanol, 1-Heptadecanol, 1-Octadecanol, 1-Eicosanol, 1-Docosanol, 1-Tetracosanol, 1-Hexacosanol, 1-Octacosanol, 1-Triacontanol, *cis*-9-Hexadecen-1-ol, *cis*-9-Octadecen-1-ol, *trans*-9-Octadecen-1-ol, *cis*-11-Octadecen-1-ol, 6,9,12-Octadecatrien-1-ol, derivaties and mixtures thereof.

The hair can be dried inbetween the application of steps (a) and (b) and the drying time is 1 to 60 minutes, wherein a drying device is used for drying the hair.

These formulations should be left on the hair for 1 to 45 minutes.

It is of advantage that the formulations of steps (a) and (b) are independently mixed into a cosmetically acceptable carrier and where the cosmetically acceptable carrier of the formulation of step (a) is either identical or not to the cosmetically acceptable carrier of the formulation of step (b).

It is also of advantage that the formulation of step (a) is mixed into a commercially available hair coloring or hair bleaching formulation prior to the application to hair.

It is also advisable that prior to the application of step (a) the hair is treated with a thioglycolic acid containing hair care composition for permanent wave treatment.

### SUMMARY OF THE INVENTION

Described herein is a method for strengthening and/or repairing hair comprising the steps of: (a) applying to the hair a formulation comprising a crosslinking composition comprising an at least bi-functional Bronsted-base of the general formulation X-R-Y, wherein X and Y are proton-acceptor groups and R is an organic spacer comprising 1 to 20 carbon atoms, and 0 to 5 oxygen atoms, and 0 to 5 nitrogen atoms, and X-R-Y having a molecular weight of less than 500 g/mol, wherein the at least bi-functional Bronsted-base is present in a concentration range from 0.1Gew.% to 25Gew.%, and leaving the hair strengthening composition on for 1 to 45 minutes, (b) optionally rinsing, shampoo'ing and/or drying the hair drying, (c) applying to the hair a hair care composition comprising an at least bi-functional Bronsted-acid which can react with the amine groups of the hair, wherein the at least bi-functional Bronsted-acid of the second formulation is present in a concentration range of 0.01Gew.% to 0.99Gew.%, and comprising at least one fatty alcohol, wherein the at least one fatty alcohol is present in a concentration of 0.1Gew.% to 25Gew.%, and leaving the hair care composition on for 1 to 45 minutes, characterized in that the first formulation has a pH of 7 to 12 and the second formulation has a pH of 1.5 to 7.

Optionally, the hair strengthening composition of step (a) can be mixed into commercially available hair coloring or hair bleaching formulations prior to the application.

### DETAILED DESCRIPTION OF THE INVENTION

In this document, including in all embodiments of all aspects of the present invention, the following definitions apply unless specifically stated otherwise. All percentages are by weight of the total composition. All ratios are weight ratios. References to `parts' e.g. a mixture of 1 part X and 3 parts Y, is a ratio by weight. "QS" or "QSP" means sufficient quantity for 100Gew.% or for 100g. +/indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 25°C and at ambient conditions, where "ambient conditions" means at 1 atmosphere (atm) of pressure and at 50Gew.% relative humidity. "Relative humidity" refers to the ratio (stated as a percent) of the moisture content of air compared to the saturated moisture level at the same temperature and pressure. Relative humidity can be measured with a hygrometer. Herein: "min" means "minute" or "minutes"; "mol" means mole; "nanometers" is abbreviated "nm"; "g" following a number means "gram" or "grams". All weights as they pertain to listed ingredients are based on the active level and do not include carriers or by-products that may be included in commercially available materials. Herein, "comprising" means that other steps and other ingredients can be in addition. "Comprising" encompasses the terms "consisting of" and "consisting essentially of". The compositions, formulationtions, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. Where amount ranges are given, these are to be understood as being the total amount of said ingredient in the composition, or where more than one species fall within the scope of the ingredient definition, the total amount of all ingredients fitting that definition, in the composition. For example, if the composition comprises from about 1Gew.% to about 5Gew.% fatty alcohol, then a composition comprising 2Gew.% stearyl alcohol and 1Gew.% cetyl alcohol, would fall within the scope.

"Viscosity" is measured at 25°C using a HAAKE Rotation Viscometer VT 550 with cooling/heating vessel and sensor systems according to DIN 53019 at a shear rate of 12.9 s⁻¹.

"Water-soluble" refers to any material that is sufficiently soluble in water to form a clear solution to the naked eye at a concentration of 0.1Gew.% by weight of the material in water at 25°C. The term "water-insoluble" refers to any material that is not "water-soluble".

"Substantially free from" or "substantially free of" means less than about 1Gew.%, or less than 0.8Gew.%, or less than 0.5Gew.%, or less than 0.3Gew.%, or about 0Gew.%, by total weight of the composition or formulationtion.

"Keratin fibres" means fibrous material composed of keratin. "Hair" means mammalian keratin fibres including scalp hair, facial hair, eyelashes, and body hair. It includes such hair still being attached to a living subject and also hair that has been removed therefrom such as hair swatches and hair on a doll/mannequin. In at least one embodiment, "hair" means human hair. "Hair shaft" or "hair fibre" means an individual hair strand and may be used interchangeably with the term "hair."

"Cosmetically acceptable" means that the compositions, formulationtions or components described are suitable for use in contact with human keratinous tissue without undue toxicity, incompatibility, instability, allergic response, and the like. All compositions and formulationtions described herein which have the purpose of being directly applied to keratinous tissue are limited to those being cosmetically acceptable.

"Derivatives" includes but is not limited to, amide, ether, ester, amino, carboxyl, acetyl, acid, salt and/or alcohol derivatives of a given compound. In at least one embodiment, "derivatives thereof" means the amide, ether, ester, amino, carboxyl, acetyl, acid, salt and alcohol derivatives.

"Monomer" means a discrete, non-polymerized chemical moiety capable of undergoing polymerization in the presence of an initiator or any suitable reaction that creates a macromolecule e.g. such as polycondensation, polyaddition, anionic or cationic polymerization. "Unit" means a monomer that has already been polymerized i.e. is part of a polymer.

"Polymer" means a chemical formed from the polymerization of two or more monomers. The term "polymer" shall include all materials made by the polymerization of monomers as well as natural polymers. Polymers made from only one type of monomer are called homopolymers. Herein, a polymer comprises at least two monomers. Polymers made from two or more different types of monomers are called copolymers. The distribution of the different monomers can be random, alternating or block-wise (i.e. block copolymer). The term "polymer" used herein includes any type of polymer including homopolymers and copolymers.

"Kit" means a package comprising a plurality of components. "Kit" may be referred to as "kit-of-parts". An example of a kit is, for example, a first composition and a separately packaged second composition and optionally application instructions.

The details of the different aspects of the invention are described hereinafter.

### Description

Described herein is a method for strengthening keratin fibres while at the same time providing excellent hair feel proterties of the treated hair and superior hair coloring and hair bleaching performance when mixed into commercially available hair color and hair bleach. The method allows the achievement of a semi-permanent strengthening of the hair shaft combined with excellent hair feel properties either upon treatment of the hair with such hair strengthening composition or upon mixing of the hair strengthening composition with commercially available hair coloring or bleaching compositions. The method comprises two distinctively different formulationtions. The first formulation has a pH value of > 7 and the second formulationtion has a pH value of < 7. The first formulation is referred to as "caustic", the second formulation as "acidic". The hair strengthening effect is retained after at least one shampoo treatment. In addition, the inventors have found that this method increases the water- and humidity-resistance of the shape, increases the ease of style and/or increases the manageability of the shape after shampooing and, when mixed with a commercially available hair coloring or bleaching formulationtion, the "caustic" hair strengthening formulationtion has no adverse effect on the hair coloring or bleaching performance itself.

Without wishing to be bound by any theory, it is believed that the above benefits are due to the steps conducted, their sequence, as well as the specific components used including the active agent and their usage concentration. It is believed that the selected active agents of the caustic formulation diffuse into the shaft of the keratin fibre, react with acid groups in the keratin polypeptide and crosslink these functional groups in the keratin protein structure, providing sufficient crosslinks to overcome the innate restoring force of the keratin fibre structure. It is also believed that the selected active agents of the acidic formulation diffuse into the shaft of the keratin fibre, react with the amino acid groups in the keratin polypeptide and crosslink these functional groups. This results in a durable strengthening of keratin fibres, for example a durable hair damage repair. As the pH value of the first formulation is caustic it mixes very well with commercially available hair coloring and hair bleaching formulationtions without deteriorating throse product's performances. As the pH value of the second formulationtion is pH < 7, hair-swelling in the alkaline pH region is reverted. Hair-swelling in the alkaline leads to the hair cuticular opening and extending away from the hair shaft which is commonly noticed as an increase in hair roughness, and is therefore undesired. It is also believed that the concentration of the active agents of the acidic formulationtion has to be < 1Gew.% to provide optimum hair feel performance. Without wanting to be bound by theory, it is believed that a low concentration of the at least bi-functional Bronsted-acid of the acidic hair strengthening composition is essential for the at least one fatty alcohol to effectively deposit on the surface of hair, even if the pH value is buffered to a defined pH value.

The details of the different aspects of the invention are described hereinafter.

### Crosslinking composition

The crosslinking compositions of the present invention are distinctively different to one another and work synergistically. The order in which they are applied is important for the benefit to be achieved. To achieve the desired hair strengthening effect in combination with excellent hair feel properties it is important that the caustic formulation is applied first and the acidic composition thereafter. Applying alkaline composition to hair leads the hair to swell. Upon swelling, the hair cuticles lift and extend away from the hair shaft causing a rough hair feel, reduced hair smoothness and less hair shine as hair fibers do not align and therefore do not provide a uniform surface to reflect light. Applying the acidic hair strengthening formulationtion after the caustic hair strengthening composition has been applied minimizes the need to formulationte additional hair smoothening actives into such hair care formulationtion which are often perceived as heavy, greasy and unnatural.

Commercial hair coloring and hair bleaching formulationtions comprise a pH adjusting agent which renders their pH caustic. In case of oxidative hair coloring, a shift in > 0.5 pH-units can result in a color shift of the final hair color. In case a commercially available hair bleaching formulationtion is applied, a shift in pH > 0.5 units reduces the bleaching power of such commercially available hair bleaching formulationtion. Therefore, if additives are added to commercially available hair coloring or hair bleaching formulationtions it is important that these additives do not alter the hair coloring or hair bleaching pH. As both, commercially available hair coloring as well as commercially available hair bleaching formulationtions have a pH of 8 to 12 it is important that those are mixed with the "caustic" formulation of the present invention. The pH value of the first hair strengthening composition is 7 to 12 to ensure no color shift when mixed with commercially available hair color or reduction in bleaching power when mixed with commercially available hair bleach products.

The acidic hair strengthening composition (b) is applied after the caustic hair strengthening composition (a) is and comprises at least bi-functional Bronsted-acid as crosslinking agents and at least one fatty alcohol and has a range of pH 1.5 to 7. The at least bi-functional Bronsted-acid can cross-link the amino groups of the hair polypeptide. Applying the acidic hair strengthening formulationtion after the caustic hair strengthening composition has been applied minimizes the need to formulationte additional hair smoothening actives into such hair care formulationtion which are often perceived as heavy, greasy and unnatural. The concentration of the at least bi-functional Bronsted-acid is from 0.01Gew.% to 0.99Gew.%. The concentration of the at least one fatty alcohol is from 0.1Gew.% to 25Gew.%, preferably from 0.5Gew.% to 15Gew.%, more preferably from 1Gew.% to 10Gew.%, ecen more preferably from 2Gew.% to 7.5Gew.%, most preferably from 3Gew.% to 5Gew.%. The at least one fatty alcohol is selected from the group of 1-Hexanol, 1-Heptanol, 1-Octanol, 1-Decanol, 1-Dodecanol, 1-Tetradecanol, 1-Hexadecanol, 1-Heptadecanol, 1-Octadecanol, 1-Eicosanol, 1-Docosanol, 1-Tetracosanol, 1-Hexacosanol, 1-Octacosanol, 1-Triacontanol, *cis*-9-Hexadecen-1-ol, *cis*-9-Octadecen-1-ol, *trans*-9-Octadecen-1-ol, *cis*-11-Octadecen-1-ol, 6,9,12-Octadecatrien-1-ol.

A dwell time of 1-45 minutes between the application steps is advisable. Optionally, the hair is rinsed and dried between the first and the second application step. Optionally, a 1-45 minutes waiting time is employed inbetween the application of the acidic and caustic hair strengthening composition.

The at least bi-functional Bronsted-base is present in a concentration of 0.1 to 25Gew.% by weight of the total composition, preferably from 0.5 to 25Gew.%, more preferably from 1 to 20Gew.%, even more preferably from 2Gew.% to 15Gew.%, and most preferably from 3 to 10Gew.%

In at least one embodiment, the acidic as well as the caustic crosslinking composition independently comprise a cosmetically acceptable carrier. In at least one embodiment, the cosmetically acceptable carrier is any carrier suitable for formulationting the active agent into a crosslinking composition being suitable for application onto hair. In at least one embodiment, the cosmetically acceptable carrier is selected from either an aqueous medium or an aqueous-alcoholic medium. In at least one embodiment, when the carrier is an aqueous-alcoholic carrier, this carrier comprises water and an alcohol. In at least one embodiment, the alcohol is selected from the group consisting of: ethanol, isopropanol, propanol, and mixtures thereof. In at least one embodiment, when the carrier is an aqueous carrier, this carrier consists essentially of water and is substantially free of alcohol. In at least one embodiment, the acidic as well as the caustic crosslinking composition independently comprise a safe and effective amount of cosmetically acceptable carrier. In at least one embodiment, the acidic as well as the caustic crosslinking composition independently comprises from about 0.1Gew.% to about 99Gew.%, or from about 1 Gew. % to about 98Gew.%, or from about 10Gew.% to about 97Gew.%, or from about 30Gew.% to about 95Gew.% water, by weight of the crosslinking composition.

Other ingredients may be present in the acidic as well as the caustic crosslinking composition. In at least one embodiment, the acidic as well as the caustic crosslinking composition comprises antioxidants. Antioxidants are useful in view of providing longer-term stability for the crosslinking composition. In at least one embodiment, the acidic as well as the caustic crosslinking composition comprises a safe and effective amount of an antioxidant. In at least one embodiment, the acidic as well as the caustic crosslinking composition comprises from about 0.001Gew.% to about 5Gew.%, or from about 0.5Gew.% to about 1.0Gew.% antioxidant. In at least one embodiment, the antioxidant is selected from the group consisting of: ascorbic acid (vitamin C), ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, peroxides including hydrogen peroxide, perborate, thioglycolates, persulfate salts, 6-hydroxy-2,5,7,8-tetramethylchroman-2-carboxylic acid (commercially available under the tradename Trolox^{™}), gallic acid and its alkyl esters, especially propyl gallate, uric acid and its salts and alkyl esters, ferulic acid and its salts and esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, 1-methionine, proline, superoxide dismutase, silymarin, tea extracts, grape skin and/or grape seed extracts, melanin, rosemary extracts, and mixtures thereof. In at least one embodiment, the antioxidant is tocopherol sorbate or an ester of tocopherol. In at least one embodiment, the antioxidant is sodium benzoate. In at least one embodiment, the antioxidant is ferulic acid. Ferulic acid has the benefit of enhancing the oxidative stability of the formulationtion. In at least one embodiment, the acidic as well as the caustic crosslinking composition comprises a safe and effective amount of ferulic acid. In at least one embodiment, the crosslinking composition comprises from about 0.001Gew. % to about 5Gew.%, or from about 0.5Gew.% to about 1.0Gew.% ferulic acid.

In at least one embodiment, the acidic as well as the caustic crosslinking composition comprises a chelator or chelating agent. As used herein, "chelator" or "chelating agent" means an active agent capable of removing a metal ion from a system by forming a complex so that the metal ion cannot readily participate in or catalyze chemical reactions. The inclusion of a chelating agent is especially useful for providing protection against UV radiation which can contribute to excessive scaling or skin texture changes and against other environmental agents which can cause skin damage, in order to decrease the local iron load, which generates, as indicated above, a pro-oxidant situation and pigmentation. A chelating agent is useful in view of providing longer-term stability for the crosslinking composition. In at least one embodiment, the first as well as the second crosslinking composition comprises a safe and effective amount of a chelator or chelating agent. In at least one embodiment, the acidic as well as the caustic crosslinking composition comprises a chelating agent, and wherein the chelating agent is selected from the group consisting of: N-hydroxysuccinimide, EDTA, NTA, deferoxamine, hydroxamic acids and their salts, phytic acid, phytate, gluconic acid and its salts, transferrine, lactoferrin, and mixtures thereof. In at least one embodiment, the acidic as well as the caustic crosslinking composition comprises a safe and effective amount of chelating agent. In at least one embodiment, the acidic as well as the caustic crosslinking composition comprises from about 0.001Gew.% to about 10Gew.%, or from about 0.01Gew.% to about 5Gew.%, or from about 0.1Gew.% to about 5Gew.%, or from about 0.5Gew.% to about 1.0Gew.% chelating agent. Exemplary chelators that are useful herein are disclosed in U.S. Pat. No. 5,487,884, issued Jan. 30, 1996 to Bissett et al.; International Publication No. 91/16035, Bush et al., published Oct. 31, 1995; and International Publication No. 91/16034, Bush et al., published Oct. 31, 1995. In at least one embodiment, the chelating agent is selected from the group consisting of: N-hydroxysuccinimide deferoxamine, lactoferrin, hydroxamic acids, gluconic acid, phytic acid, derivatives thereof, and mixtures thereof.

In at least one embodiment, the acidic as well as the caustic crosslinking composition is in a form suitable for application onto hair. In at least one embodiment, the acidic as well as the caustic crosslinking composition is in the form of an emulsion, a solution, or a dispersion. In at least one embodiment, the crosslinking composition comprises a surfactant. The surfactant can be useful in providing an emulsion. In at least one embodiment, when being in the form of an emulsion, said emulsion may be a water-in-oil emulsion, an oil-in-water emulsion, or a multiple emulsion. An emulsion has the benefit of providing an easy-to-apply composition for the consumer to apply to the hair and has aesthetic advantages. The acidic as well as the caustic crosslinking composition may be a leave-in composition or a rinse-off composition. The acidic as well as the caustic crosslinking composition may be the form of a hair conditioning composition.

The acidic as well as the caustic crosslinking composition may further comprise at least one cosmetic agent selected from hairstyling polymers, conditioning agents, hair cleansing agents, or mixtures thereof. In at least one embodiment, the acidic as well as the caustic crosslinking composition comprises a hairstyling polymer. In at least one embodiment, the hairstyling polymer is selected from the group consisting of: non-ionic hairstyling polymer, anionic hairstyling polymer, zwitterionic and/or amphoretic hairstyling polymer, cationic hair styling polymer, or mixtures thereof. Suitable hairstyling polymers may be found in the CTFA International Cosmetics Ingredient Dictionary and Handbook, "Hair Fixatives", 12th edition (2008). Suitable hairstyling polymers are, for example, those materials disclosed from page 12, line 5 to page 19, line 1 of the European patent application 08151246.9 filed on 11 February 2008, which is incorporated herein by reference.

In at least one embodiment, the acidic as well as the caustic crosslinking composition comprises from about 0.01Gew.% to about 10Gew.% by weight, or from about 0.1Gew. % to about 8Gew.%, or from about 0.1Gew.% to about 5Gew.% hairstyling polymer.

In at least one embodiment, the crosslinking compositions comprise a non-ionic hairstyling polymer. In at least one embodiment, the non-ionic hairstyling polymer is a natural or synthetic polymer. In at least one embodiment, the non-ionic hair styling polymers is a polymer obtained from the polymerization of at least one type of monomer selected from: vinylpyrrolidone; vinylcaprolactam; vinyl esters; vinyl alcohol; vinyl acetate; (meth)acrylamide, and/or its derivatives; (meth)acrylic acid, its salts, and/or its derivatives; propylene and/or ethylene glycol acid; crotonic acid; or mixtures thereof. For example, such polymers are available under the trade names Luviskol^{®} or Luviset Clear^{®}.

In at least one embodiment, the crosslinking compositions comprise an anionic hairstyling polymer. In at least one embodiment, the anionic hairstyling polymer is selected from the group consisting of: acrylic acid/alkyl acrylate/Nalkylacrylamide terpolymer; vinyl acetate/crotonic acid copolymer; C1-C5-alkyl acrylate/(meth)acrylic acid copolymer; sodium polystyrenesulfonate; vinyl acetate/crotonic acid/vinyl alkanoate copolymer; vinyl acetate/crotonic acid/vinyl neodecanoate copolymer; aminomethylpropanol acrylate copolymer; vinylpyrrolidone/(meth)acrylic copolymer; methyl vinyl ether/maleic monoalkyl esters copolymer; aminomethylpropanol salts of allyl methacrylate/(meth)acrylate copolymer; ethyl acrylate/methacrylic acid copolymer; vinyl acetate/mono-nbutyl maleate/isobornyl acrylate copolymer; octylacrylamid/(meth)acrylic acid copolymer; polyesters of diglycol, cyclohexanedimethanol, isophthalic acid and sulfoisophthalic acid; and mixtures thereof.

In at least one embodiment, the acidic as well as the caustic crosslinking composition comprises a zwitterionic or amphoteric hairstyling polymer. In at least one embodiment, the zwitterionic or amphoteric hairstyling polymer is selected from the group consisting of: alkylacrylamide/alkylaminoalkyl methacrylate/(meth)acrylic acid copolymers; copolymers which are formed from at least one first monomer type which has quaternary amine groups, and at least one second monomer type which has acid groups; copolymers of fatty alcohol acrylates, of alkylamine oxide methacrylate and at least one monomer chosen from acrylic acid and methacrylic acid; methacryloylethylbetaine/methacrylic acid and/or esters copolymers; polyquaternium-47; polyquaternium-43; oligomers or polymers, preparable from quaternary croton betaines or quaternary croton betaine esters; or mixtures thereof.

In at least one embodiment, the acidic as well as the caustic crosslinking composition comprises a cationic hairstyling polymer. In at least one embodiment, the cationic hairstyling polymer is selected from the group consisting of homopolymers or copolymers where a quaternary nitrogen groups are present either in the polymer chain or as substituent on one or more of the cationic monomers. The monomers containing ammonium groups may be copolymerized with non-cationic monomers. Suitable cationic monomers may be unsaturated, free-radically polymerizable compounds which carry at least one cationic group, in particular ammonium-substituted vinyl monomers, such as, for example, trialkylmethacryloxyalkylammonium, trialkylacryloxyalkylammonium, dialkyldiallylammonium and quaternary vinylammonium monomers with cyclic, cationic nitrogen-containing groups, such as pyridinium, imidazolium or quaternary pyrrolidones, e.g. alkylvinylimidazolium, alkylvinylpyridinium, or alkylvinylpyrrolidone salts. The alkyl groups of these monomers are preferably lower alkyl groups, such as, for example, C1 to C7-alkyl groups, particularly preferably C1 to C3-alkyl groups. Suitable non-cationic monomers may be selected from (meth)acrylamide, derivatives thereof; acrylate, its derivative thereof; vinylcaprolactone, vinylcaprolactam, vinylpyrrolidone, vinyl esters, vinyl alcohol, propylene glycol or ethylene glycol. For example, suitable cationic hairstyling polymers are available under the tradenames Gafquat 755 N; Gafquat 734; Gafquat HS 100; Luviquat HM 550; Merquat Plus 3300; Gaffix VC 713; Aquaflex SF 40. In at least one embodiment, the crosslinking compositions comprise a cationic hairstyling polymer derived from a natural polymer. In at least one embodiment, the cationic hairstyling polymer derived from a natural polymer is derived from a natural polymer selected from the group consisting of: cationic derivatives polysaccharides such as cellulose, starch and/or guar; chitosan, its salts, and/or its derivatives; or mixtures thereof. In at least one embodiment, the cationic hairstyling polymers are selected from the group consisting of: polyquaternium-4; polyquaternium-10; polyquaternium-24; guar hydroxypropyltrimonium chloride; chitosonium pyrrolidonecarboxylate; and mixtures thereof.

In at least one embodiment, the acidic as well as the caustic crosslinking composition comprises a conditioning agent, or a hair conditioning agent. The acidic as well as the caustic crosslinking composition may comprise any suitable and conventional hair conditioning agents. The term "hair conditioning agent" herein means any cosmetically acceptable compound having a cosmetic effect on hair, such as providing gloss to hair, making hair more manageable, improving hair touch, improving combability and/or giving hair more volume. Suitable hair conditioning agents may be found in the CTFA International Cosmetics Ingredient Dictionary and Handbook, "Hair conditioning agents", 12th edition (2008). In at least one embodiment, the hair conditioning agent is selected from the group consisting of: cationic surfactants, non-ionic surfactants, silicone compounds, organic oily conditioning agents, and mixtures thereof. Suitable hair conditioning agents are, for example, those materials disclosed from page 19, line 3 to page 27, line 33 of the European patent application 08151246.9 filed on 11 February 2008, which is incorporated herein by reference.

In at least one embodiment, the conditioning agent is a cationic surfactant. In at least one embodiment, the cationic surfactant comprises amino or quaternary ammonium moieties. In at least one embodiment, the acidic as well as the caustic crosslinking composition comprises from about 0.05Gew.% to about 3.5Gew.%, or from about 0.1Gew.% to about 3.0Gew.%, or from about 0.5Gew.% to about 2.5Gew.%, or from about 1.0Gew.% to about 2.0Gew.% cationic surfactant. In at least one embodiment, the cationic surfactant is according to Formulation II: 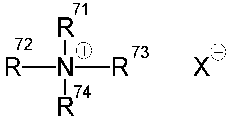 wherein at least one of R⁷¹, R⁷², R⁷³ and R⁷⁴ is selected from: an aliphatic group of from 8 to 30 carbon atoms; an aromatic, alkoxy, polyoxyalkylene, alkylamido, hydroxyalkyl, aryl; or an alkylaryl group having from 7 to 22 carbon atoms; wherein the remainder of R⁷¹, R⁷², R⁷³ and R⁷⁴ are independently selected from the group consisting of: an aliphatic group consisting of from 1 to 22 carbon atoms; and an aromatic, alkoxy, polyoxyalkylene, alkylamido, hydroxyalkyl, aryl or alkylaryl group having up to 22 carbon atoms; wherein X is selected from the group consisting of: halogen, acetate, citrate, lactate, glycolate, phosphate, nitrate, sulfonate, sulfate, alkylsulfate, alkyl sulfonate radicals, and mixtures thereof. In at least one embodiment, cationic surfactant is according to Formulation II (see above), wherein at least one of R⁷¹, R⁷², R⁷³ and R⁷⁴ is an aliphatic group having from 16 to 24 carbon atoms; wherein the remainder of R⁷¹, R⁷², R⁷³ and R⁷⁴ are independently selected from the group consisting of aliphatic groups having from 1 to 4 carbon atoms; wherein X is selected from the group consisting of: chloride or sulfate. In at least one embodiment, the cationic surfactant is selected from the group consisting of: behenyltrimethylammonium chloride, methyl sulfate or ethyl sulfate; stearyltrimethylammonium chloride, methyl sulfate or ethyl sulfate; and mixtures thereof. It is believed that a longer alkyl group provides improved smoothness and soft feeling on wet and dry hair, compared to cationic surfactants with a shorter alkyl group. It is also believed that such cationic surfactants can provide reduced scalp irritation, compared to those having a shorter alkyl group. In at least one embodiment, the cationic surfactant is a di-long alkyl quaternized ammonium salt selected from the group consisting of: dialkyl (14-18 carbons) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, and mixtures thereof. In at least one embodiment, the cationic surfactant is a tertiary amidoamine having an alkyl group of from about 12 to about 22 carbons. In at least one embodiment, the cationic surfactant is selected from the group consisting of: cetyl trimethyl ammonium salts; behenyl trimethyl ammonium salts; dimethyl ditallow ammonium salts; stearyl amidopropyl dimethylamine; (di)esterquats; quaternium 8, 14, 15, 18, 22, 24, 26, 27, 30, 33, 37, 53, 60, 61, 72, 78, 80, 81, 82, 83, 84, and/or 91; or mixtures thereof.

In at least one embodiment, the conditioning agent is a non-ionic surfactant. Suitable non-ionic surfactants may be surfactants having a HLB of less than 8. Suitable nonionic surfactants may be selected from glyceryl esters; sugar esters; alkylpolyglucoside ethers; oleyl- or isostearylpolyglucoside; polyoxyethylene (20) sorbitan monostearate; or mixtures thereof.

In at least one embodiment, the conditioning agent is a silicone compound. In at least one embodiment, the silicone compound is volatile or nonvolatile, and/or soluble or insoluble silicones. For example, suitable silicone conditioning agents are available under the tradenames SF 1075 methyl phenyl fluid (Electric company); DC200 Fluid, DC244, DC245, DC345, Dow 5-7113, DC556 Cosmetic Grade Fluid, DC1248 (Dow Corning). In at least one embodiment, the acidic as well as the caustic crosslinking composition comprises a conditioning agent being the reaction product of: (a) an aminosilane; (b); polysiloxane; and optionally (c) a polyether. In at least one embodiment, the acidic as well as the caustic crosslinking composition comprises a conditioning agent being the reaction product of: (a) an aminosilane; (b); polysiloxane; and (c) a polyether. In at least one embodiment, the acidic as well as the caustic crosslinking composition comprises a conditioning agent, and wherein the conditioning agent is selected from the group consisting of: epoxyaminosilane copolymers, and polysiloxane/polyurea block copolymers, and mixtures thereof. In at least one embodiment, the acidic as well as the caustic crosslinking composition comprises a conditioning agent being the reaction product of: (a) an aminosilane; (b) polysiloxane; and (c) a polyether; and optionally (d) an amine. In at least one embodiment, the polysiloxane is an epoxy encapped polysiloxane. In at least one embodiment, the polysiloxane comprises at least two oxirane or oxetane groups. In at least one embodiment, the polysiloxane comprises from about 10 to about 450 silicon atoms, or from about 40 to about 400 silicon atoms, from about 75 to about 350 silicon atoms, from about 150 to about 250 silicon atoms. In at least one embodiment, the polysiloxane is an epoxy encapped polysiloxane. In at least one embodiment, the polyether has the structure CH₂(O)CHCH₂O(CH₂(CH₃)CH₂O)ₙCH₂CH(O)CH₂ (average) wherein n is an integer from 1 to 10. In at least one embodiment, the amine comprises from 1 to 10 carbon atoms, or from 2 to 5 carbon atoms. In at least one embodiment, the amine is an alkylamine that is substituted with at least one alkyl group. In at least one embodiment, the amine is selected from the group consisting of: methylamine, ethylamine, propylamine, ethanol amine, isopropylamine, butylamine, isobutylamine, hexylamine, dodecylamine, oleylamine, aniline aminopropyltrimethylsilane, aminopropyltriethylsilane, aminomorpholine, aminopropyldiethylamine benzylamine, napthylamine 3-amino-9-ethylcarbazole, 1-aminoheptaphlorohexane, 2,2,3,3,4,4,5,5,6,6,7,7,8,8,8-pentadecafluoro-1-octanamine, and mixtures thereof. In at least one embodiment, the amine is selected from the group consisting of: methylethylamine, methylhexylamine, methyloctadecylamine, diethanolamine, dibenzylamine, dihexylamine dicyclohexylamine, piperidine, pyrrolidine phthalimide, and mixtures thereof. In at least one embodiment, the conditioning agent is an epoxyaminosilane copolymer. In at least one embodiment, the conditioning agent is conditioning agent being the reaction product of: (a) an aminosilane; (b) polysiloxane, wherein the polysiloxane comprises from about 10 to about 450 silicon atoms, or from about 40 to about 400 silicon atoms; and (c) a polyether; and (d) an amine, wherein the amine is an alkylamine that is substituted with at least one alkyl group.

In at least one embodiment, the acidic as well as the caustic conditioning agent is selected from the group consisting of: epoxyaminosilane copolymers, and polysiloxane/polyurea block copolymers, and mixtures thereof. In at least one embodiment, the conditioning agent is a polydimethylsiloxane-derivative comprising aminoalkyl groups and having an amine number of at least 0.1 meq/g of polydimethylsiloxane. Such polydimethylsiloxane-derivative can, for example, be methoxy-terminated or hydroxy-terminated, or mixtzures thereof.

In at least one embodiment, the conditioning agent is an organic oily conditioning agent. In at least one embodiment, the organic oily conditioning agent is non-volatile, water-insoluble, oily or fatty. Organic oily conditioning agents may be selected from hydrocarbon oils and fatty esters.

The the acidic as well as the caustic crosslinking composition may further comprise at least one direct hair dye. In at least one embodiment, the crosslinking compositions comprises from about 0.01Gew.% to about 15Gew.%, or from about 0.1Gew.% to about 10 Gew.%, or from about 0.5Gew.% to about 8Gew.% direct hair dye.

The the acidic as well as the caustic crosslinking composition may further comprise at least one viscosity-modifying agent. In at least one embodiment, the crosslinking compositions comprise from about 0.01Gew.% to about 20Gew.%, or from about 0.05Gew.% to about 10Gew.%, or from about 0.1Gew.% to about 5Gew.% viscosity-modifying agent.

The the acidic as well as the caustic crosslinking composition may further comprise at least one emulsifier and/or surfactant. In at least one embodiment, the emulsifier and/or surfactant is selected from nonionic surfactants; anionic surfactants; amphoretic surfactants; or mixtures thereof. In at least one embodiment, the the acidic as well as the caustic crosslinking composition comprises from about 0.01Gew.% to about 20Gew.%, or from about 0.05Gew.% to about 10Gew.%, or from about 0.1Gew.% to about 5Gew.%, emulsifier and/or surfactant.

The acidic as well as the caustic crosslinking composition may further comprise at least one pigment. In at least one embodiment, the pigment is selected from natural pigments; synthetic pigments; or mixtures thereof. The pigments may be selected from organic pigment, inorganic pigment; or mixtures thereof. The pigments may be selected from colored pigments; pearlescent pigments; or mixtures thereof. Said acidic as well as the caustic crosslinking composition may comprise from about 0.01Gew.% to 10Gew.%, or from about 1Gew.% to about 2Gew.% pigment present in the product mass in undissolved form by weight of the total composition. The acidic as well as the caustic crosslinking composition may comprise pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water-soluble components such as those having C.I. Names.

In at least one embodiment, the acidic as well as the caustic crosslinking composition comprises at least one particulate substance. In at least one embodiment, the particulate substance is selected from silica; silicates; aluminates; clay earths; mica; insoluble salts, particularly insoluble inorganic metal salts; metal oxides; minerals; insoluble polymer particles; or mixtures thereof. In at least one embodiment, the acidic as well as the caustic crosslinking composition comprises from about 0.01Gew. % to about 10Gew.%, or from about 0.05Gew.% to about 5Gew.% of at least one particulate substance. In at least one embodiment, the acidic as well as the caustic crosslinking composition is substantially free of a particulate substance such as clay.

In at least one embodiment, the acidic as well as the caustic crosslinking composition comprises at least one preservative. In at least one embodiment, the acidic as well as the caustic crosslinking composition may comprise from about 0.01Gew. % to about 5Gew.% by weight, or from about 0.05Gew.% to about 1Gew.% preservative.

A variety of additional optional ingredients may be incorporated into the acidic as well as the caustic crosslinking composition of the present invention. Nonlimiting examples of these additional ingredients may be selected from preservatives; antioxidants; sequestering agents; solvents; fragrances & perfumes; fillers; screening agents; odor absorbers; coloring materials; lipid vesicles; detersive surfactants; thickening agents and suspending agents; viscosity modifiers; pearlescent aids; UV-filters and sunscreens; agents for combating free radicals; polyvinyl alcohol; pH adjusting agents; salts; coloring agents; polymer plasticizing agents; direct dyes; or mixtures thereof. The acidic as well as the caustic crosslinking composition may comprise from about 0Gew.%, or from about 0.1Gew.% to about 5Gew.% antimicrobial agents. In at least one embodiment, the acidic as well as the caustic crosslinking composition comprises an organic acid selected from the group consisting of: glycine, L-methionine, L-arginine, biotin, creatine, and mixtures thereof. In at least one embodiment, the acidic as well as the caustic crosslinking composition comprises panthenol. In at least one embodiment, the acidic as well as the caustic crosslinking composition comprises a wax compound. In at least one embodiment, the acidic as well as the caustic crosslinking composition comprises beeswax.

In at least one embodiment, the acidic as well as the caustic crosslinking composition has a viscosity, measured at 25°C, of from about 0.1 mPa·s to about 1,000,000 mPas, or from about 1 mPa·s to about 80,000 mPa s, or from about 5 mPa·s to about 3,500 mPa s. The viscosity is measured by HAAKE Rotation Viscometer VT 550 with cooling/heating vessel and sensor systems according to DIN 53019 (MV-DIN, SV-DIN), shear rate is 12.9 s⁻¹.

In at least one embodiment, the acidic as well as the caustic crosslinking composition comprises a buffering agent. In at least one embodiment, the buffering agent is a phosphate buffer. In at least one embodiment, the buffering agent is a borate buffer or a carbonate buffer. In at least one embodiment, the buffering agent is selected from the group consisting of: glycine/sodium hydroxide; sodium carbonate/sodium hydrogen carbonate, sodium tetraborate/sodium hydroxide; sodium bicarbonate/sodium hydroxide; ammonium chloride/ammonia. The buffering agent has the advantage of controlling the pH, which aids the stability of the crosslinking compositions. In at least one embodiment, the caustic crosslinking composition comprises an alkalizing agent. Such alkalizing agent can be organic or inrorganic and can be mono-functional or poly-functional. In at least one embodiment, the acidic crosslinking composition comprises an acidifying agent. Such acidifying agent can be organic or inrorganic and can be mono-functional or poly-functional. In at least one embodiment, such acidifying agent of the acidic formulation is identical to the at least bi-functional Brønsted-acid of the caustic formulation.

### Method

Described herein is a hair strengthening and/or hair repairing method comprising: (a) applying a caustic crosslinking composition to hair and leaving the caustic hair strengthening composition on for 1 to 45 minutes; (b) optionally rinsing, shampoo'ing and/or drying the hair drying; (c) applying an acidic hair strengthening composition to hair and leaving the acidic hair strengthening composition on for 1 to 45 minutes; (d) optionally rinsing, shampoo'ing and/or drying the hair drying. Prior to step (a) the caustic hair strengthening composition can be mixed with commercially available hair coloring or bleaching formulationtions. In the method of the present invention, the acidic as well as the caustic crosslinking composition may be applied on wet hair and/or on dry hair.

In an embodiment, prior to the first step of the method the hair is washed with a shampoo, for example a cleansing shampoo. In an embodiment, at the end of the treatment cycle the hair is conditioned with a conditioning formulationtion comprising a conditioning agent. Conditioning agents are disclosed herein and are suitable for this embodiment. In an embodiment, the hair is dried using a blow dryer and a brush.

In an embodiment, the method relates to a hair strengthening and/or hair repairing method comprising: (a) applying to the hair a formulation comprising a crosslinking composition comprising an at least bi-functional Bronsted-base of the general formulation X-R-Y, wherein X and Y are proton-acceptor groups and R is an organic spacer comprising 1 to 20 carbon atoms, and 0 to 5 oxygen atoms, and 0 to 5 nitrogen atoms, and X-R-Y having a molecular weight of less than 500 g/mol, wherein the at least bi-functional Bronsted-base is present in a concentration range from 0.1Gew.% to 25Gew.%, and leaving the hair strengthening composition on for 1 to 45 minutes, (b) optionally rinsing, shampoo'ing and/or drying the hair drying, (c) applying to the hair a hair care composition comprising an at least bi-functional Bronsted-acid which can react with the amine groups of the hair, wherein the at least bi-functional Bronsted-acid of the second formulation is present in a concentration range of 0.01Gew.% to 0.99Gew.%, and comprising at least one fatty alcohol, wherein the at least one fatty alcohol is present in a concentration of 0.1Gew.% to 25Gew.%, and leaving the hair care composition on for 1 to 45 minutes, characterized in that the first formulation has a pH of 7 to 12 and the second formulation has a pH of 1.5 to 7.

Optionally, the crosslinking composition of step (a) can be mixed into commercially available hair coloring or hair bleaching formulationtions prior to the application.

### Applying a hair care composition to hair

The present invention relates to a hair strengthening and/or repairing method comprising: (a) applying a caustic hair care composition to hair, wherein the caustic hair care composition comprises an at least bi-functional Bronsted-base capable of reacting with the carboxylic acid groups of the hair, involving applying onto hair from about 0.01 gram to about 5 gram of said compositions per gram hair, and (c) applying to the hair an acidic hair care composition comprising an at least a bi-functional Bronsted-acid which can react with the amine groups of the hair and comprising at least one fatty alcohol. In an embodiment, the acidic as well as the caustic composition are on the hair for at least 1 min, or from about 5 min to about 45 min, or from about 10 min to about 40 min, or from about 20 min to about 35 min.

### Hair drying

The hair strengthening method may optionally comprise hair drying in steps (b) and/or (d). In an embodiment, the hair drying is carried out by a blow drier. In an embodiment, the hair drying is carried out for a duration of from about 1 min to about 45 min, or from about 2 min to 20 min, or from about 5 min to 15 min. In general, following the hair drying, the hair can still be damp, but needs to have reasonable e.g. 75Gew.% hair fibre separation of the head of hair. Some residual moisture in the hair is acceptable. In an embodiment, the hair drying is carried out by a hood appliance. In an embodiment, the hair drying is carried out by toweling hair and/or by pressing hair with hands.

Hair dryer or blow dryer distances between device and head are typically down to about 10 cm. Blow dryers direct hot air through some sort of attachment for combing or otherwise treating the hair. A blow dryer is typically used such that the distance to the hair (for example at a distance of 20 or 30 or 40 centimeters) and often is used with the aid of a comb or a brush. In an embodiment, the hair drying is carried out by a blow drier at a temperature of from about from 50°C to about 100°C. In an embodiment, the hair drying is carried out by a blow drier at a temperature of up to 130°C. In an embodiment, the hair drying is carried out with a blow drier with brushing to help styling the hair.

In an embodiment, the hair strengthening and/or repairing method comprises in addition to steps (a), (b), (c), (d) also a hair straightening step (e). The hair straightening step (e) comprises using a hair straightening appliance comprises metal or ceramic plates. In an embodiment, the metal or ceramic plates are provided to a temperature of from about 100°C to about 280°C. In an embodiment, the metal or ceramic plates are provided to a temperature of from about 110°C to about 250°C, or from about 120°C to about 240°C, or from about 140°C to about 230°C, or from about 160°C to about 220°C, or from about 180°C to about 210°C, or from about 190°C to about 200°C.

In an embodiment, the 'straightening of the hair with the appliance' step is carried out for a duration of from about 1 min to about 45 min, or from about 2 min to 20 min, or from about 5 min to 15 min. In an embodiment, the 'mechanically straightening the hair with the appliance' is carried out for a duration of for at least 10 min, or for at least 12 min.

In an embodiment, method (a) to (d) is repeated from 2 to 4 times per month on an ongoing basis for the purpose of strengthening hair and reducing hair damage.

In an embodiment, the caustic crosslinking composition may comprise a first, second, and third crosslinking agent. The first crosslinking agent may be 4,7,10-Trioxa-1,13-tridecanediamine, the second may be 4,9-Dioxa-1,12-dodecanediamine, and the third crosslinking agent may be 1,11-Diamino-3,6,9-trioxaundecane. The caustic crosslinking composition can also comprises an organic or an inorganic acid to adjust the pH of the caustic crosslinking to pH 7 to 12.

In an embodiment, the acidic crosslinking composition may comprise a first, a second, and a third crosslinking agent. The crosslinking agents used in the acidic crosslinking composition are at least bi-functional organic acids. The first cross-linking agent may be Malic acid, the second may be Fumaric acid, the third may be Citric acid. The acidic crosslinking composition can also comprises an organic or an inorganic base to adjust the pH of the acidic crosslinking to pH 1.5 to 7.

In an embodiment, the caustic as well as the acidic crosslinking composition may also comprise a buffering agent, a cosmetically acceptable carrier, a conditioning agent being the reaction product of: (a) an aminosilane; (b); polysiloxane; and optionally (c) a polyether;

In an embodiment, the formulationtion comprises from about 0.01Gew.% to about 15Gew.%, or from about 0.1Gew.% to about 10Gew.%, or from about 1Gew.% to about 5Gew.% conditioning agent being the reaction product of: (a) an aminosilane; (b); polysiloxane; and optionally (c) a polyether.

In an embodiment, a kit may comprise: (i) a caustic crosslinking composition; (ii) an acidic crosslinking composition; (iii) a conditioning composition. In an embodiment, the kit may be for strengthening and repairing damaged hair. In an embodiment, the kit may be for improving ease of hair styling.

In an embodiment, the crosslinking agent may be used for strengthening hair and / or repairing damaged hair. In an embodiment, the caustic as well as the acidic crosslinking composition may be used for improving ease of styling of the hair.

### EXAMPLES

The following examples further describe and demonstrate embodiments within the scope of the present invention. The examples are given solely for the purpose of illustration, and are not to be construed as limitations of the present invention since many variations thereof are possible without departing from its scope.

### Example compositions for the caustic crosslinking composition (total mass 100g):

Liquid A: 15 g 4,7,10-Trioxa-1,13-tridecanediamine, QSP water
Liquid B: 25 g 4,9-Dioxa-1,12-dodecanediamine, QSP water
Liquid C: 5 g 1,11-Diamino-3,6,9-trioxaundecane, QSP water

### Example compositions for the acidic crosslinking composition (total mass 100g):

Composition A: 3.00 g Maleic Acid, 3.00 g Monoethanolamine, 8.00 g Cetyl Alcohol (1-hexadecanol), 1.00 g Cetyltrimethyl Ammonium Chloride, 1.00 g Polymethylphenyl Siloxane, 0.40 g Phenoxy Ethanol, 0.20 g PHB-methylester, 1.00 g Dow Corning 949 Cationic Emulsion^{®}, 5.00 g Isododecane, 0.40 g Perfume Oil, QSP water.

Composition A*: 0.75 g Maleic Acid, 0.75 g Monoethanolamine, 8.00 g Cetyl Alcohol (1-hexadecanol), 1.00 g Cetyltrimethyl Ammonium Chloride, 1.00 g Polymethylphenyl Siloxane, 0.40 g Phenoxy Ethanol, 0.20 g PHB-methylester, 1.00 g Dow Corning 949 Cationic Emulsion^{®}, 5.00 g Isododecane, 0.40 g Perfume Oil, QSP water.

Composition B: 4.00 g Malic Acid, 4.00 g Monoethanolamine, 5.00 g Cetearyl Alcohol (a mixture of fatty alcohols including 1-hexadecanol and 1-octadecanol), 1.00 g Cetyltrimethyl Ammonium Chloride, 1.00 g Polymethylphenyl Siloxane, 0.40 g Phenoxy Ethanol, 0.20 g PHB-methylester, 1.00 g Dow Corning 949 Cationic Emulsion^{®}, 5.00 g Isododecane, 0.40 g Perfume Oil, QSP water.

Composition B*: 0.80 g Malic Acid, 0.80 g Monoethanolamine, 5.00 g Cetearyl Alcohol (a mixture of fatty alcohols including 1-hexadecanol and 1-octadecanol), 1.00 g Cetyltrimethyl Ammonium Chloride, 1.00 g Polymethylphenyl Siloxane, 0.40 g Phenoxy Ethanol, 0.20 g PHB-methylester, 1.00 g Dow Corning 949 Cationic Emulsion^{®}, 5.00 g Isododecane, 0.40 g Perfume Oil, QSP water.

Composition C: 3.00 g Fumaric Acid, 3.00 g Itaconic Acid, 6.00 g Monoethanolamine, 4.00 g 1-Dodecanol, 1.00 g Cetyltrimethyl Ammonium Chloride, 1.00 g Polymethylphenyl Siloxane, 0.40 g Phenoxy Ethanol, 0.20 g PHB-methylester, 1.00 g Dow Corning 949 Cationic Emulsion^{®}, 5.00 g Isododecane, 0.40 g Perfume Oil, QSP water.

Composition C*: 0.25 g Fumaric Acid, 0.25 g Itaconic Acid, 0.50 g Monoethanolamine, 4.00 g 1-Dodecanol, 1.00 g Cetyltrimethyl Ammonium Chloride, 1.00 g Polymethylphenyl Siloxane, 0.40 g Phenoxy Ethanol, 0.20 g PHB-methylester, 1.00 g Dow Corning 949 Cationic Emulsion^{®}, 5.00 g Isododecane, 0.40 g Perfume Oil, QSP water.

### Data

The hair strengthening efficacy as well as hair feel is tested for various combinations of the caustic as well as the acidic crosslinking compositions of the present invention. Switches of low lift natural hair are employed. These are shampooed with a K-PAK clarifying shampoo to ensure the hair is in a clean state with no residues that could affect the end result. The switches are then rinsed. Excess water is removed from the hair by wringing out the switches. The switches are treated with a crosslinking compositions as listed in TABLE 1 Crosslinking compositions are left on the hair for 30 minutes. After this time, the hair is blow dried and the hair feel is assessed by 5 individuals. To assess hair feel, 5 individuals have 5 treated hair switches run through their fingers and grade those 5 switches according to a five point scale where grade 5 is assigned to swiches of optimum softness and grade 1 is assigned when hair switches are perceived rough. Results per hair switch are averaged. The same hair switches are then brushed with a standard metal comb for 500 strokes. Hair strengthening and hair damage is assed via recording of the weight of broken hair fibers collected from the combing and normalized to the weight of the hair switches. 5 hair switches per experiment are treated and combed, results are averaged. Hair switches were first treated with the caustic hair strengthening composition according to the above mentioned description without an inbetween hair drying step, then followed by the treatment of the acidic hair strengthening composition as per the above mentioned description, followed by hair drying.

**Table 1. Hair Breakage / Hair Feel results after n combing strokes**

| Treatment | 50 strokes | 250 strokes | 500 strokes | Hair Feel |
|---|---|---|---|---|
| Reference: Untreated hair | 5,0Gew.% | 8,1Gew.% | 9,7Gew.% | 1,6 |
| First: Liquid A | 3,4Gew.% | 4,7Gew.% | 6,2Gew.% | 2,8 |
| Second: Composition A | | | | |
| First: Liquid A | 3,3Gew.% | 4,7Gew.% | 6,3Gew.% | 3,6 |
| Second: Composition A* | | | | |
| First: Liquid B | 2,6Gew.% | 4,1 Gew.% | 5,8Gew.% | 2,7 |
| Second: Composition B | | | | |
| First: Liquid B | 3,0Gew.% | 4,3Gew.% | 6,1Gew.% | 3,7 |
| Second: Composition B* | | | | |
| First: Liquid C | 2,9Gew.% | 3,6Gew.% | 5,9Gew.% | 2,5 |
| Second: Composition C | | | | |
| First: Liquid C | 3,1Gew.% | 4,0Gew.% | 6,0Gew.% | 3,6 |
| Second: Composition C* | | | | |
| First: Liquid A | 3,1 Gew.% | 3,9Gew.% | 6,2Gew.% | 2,6 |
| Second: Composition B | | | | |
| First: Liquid A | 3,0Gew.% | 4,2Gew.% | 6,2Gew.% | 3,8 |
| Second: Composition B* | | | | |
| First: Liquid B | 2,6Gew.% | 3,8Gew.% | 5,9Gew.% | 2,6 |
| Second: Composition C | | | | |
| First: Liquid B | 2,72Gew. % | 3,7Gew.% | 6,0Gew.% | 3,9 |
| Second: Composition C* | | | | |

When averaging over five experimental results, the relative standard deviation is less than 15Gew.%. As can be seen in Table 1, the sequential application of a caustic hair strengthening composition of the present invention with an acidic hair strengthening composition of the present invention significantly reduces hair breakage. As can also be seen from Table 1, the hair feel properties of the hair switches significantly improve when the amount of the acidic hair strengthening agents are reduced. Without wanting to be bound by theory, it is believed that the amount of the acidic hair strengthening agent used affects the way the fatty alcohols distribute on the surface of hair. It is believed that when the concentration of the acidic hair strengthening agent is less than 1Gew.% by total weight of the formulationtion, optimum hair properties are achievd. Same hair breakage reduction results and hair feel property improvements were achieved when the caustic hair strengthening composition was first mixed into a commercially available hair coloring formulationtion, left on the hair switch for a time specified by the hair color manufacturer's guidance, followed by applying the acidic hair strengthening composition as per instructions above, followed by subsequent hair drying. The hair coloring results are excellent. No shift in hair color is observed when the caustic hair strengthening composition is mixed into commercially available hair color. A mixing ratio of 10 g of a commercially available cream hair color with 15 g of a commercially available 6Gew.% Peroxide hair color developer and 2 g of the caustic hair strengthening composition was used.

To color human hair using oxidative dye technology it is generally necessary to treat the hair with a mixture of suitable oxidative coloring agents and at least one dye oxidizing agent. Hydrogen peroxide is the most commonly used dye oxidizing agent. However, in addition to dye oxidation, hydrogen peroxide treatment of the hair can also solubilize the colored melanin component in the hair and can lead to undesirable hair qualities, such as poor condition, due to increased brittleness and hair damage. These undesirable qualities are in part due to the necessary conditions of conventional peroxide treatment, as part of the hair coloring process, which requires high pH (>pH 9), extended exposure (from 10 to 60 minutes) and relatively high concentration of oxidizing solutions (up to 20Gew.% volume of oxygen) in order to deliver effective dye oxidization. Thus there is a need for hair coloring compositions which can oxidize dyes and color the hair effectively and, at the same time, strengthen the hair to provided prevent hair damage.

The process for hair bleaching is very similar to the process of hair coloring. Bleaching, basically, is a process of removing the natural color from hair. Because of the virtually unlimited variations of hair colors, bleaching per se, does not usually produce a uniform or aesthetically pleasing color in hair, nor will it produce a color tone other than that inherent in the hair. For these reasons hair that has been bleached is subsequently treated with a hair toner, a composition containing a hair dye which imparts the desired end-color to the bleached hair. The degree to which the natural color must be bleached from the hair is primarily determined by the desired end color. The toners do not lighten the shade of hair to any great extent; they impart their tone coloration to hair pre-bleached to the basic blonde shade desired, e.g. pastel blonde color tone is achieved in hair pro-bleached to pale blonde not in hair pre-bleached only to a light brown.

Applied by itself or mixed with commercially available hair coloring or hair bleaching formulationtions, followed by subsequent application of the acidic hair strengthening composition, treating hair with the caustic as well as the acidic hair strengthening composition of the present invention improves the quality of the hair, reduces hair breakage, reduces hair damage, improves hair feel, reduces hair roughness, improves luster and hair shine, eases hair styling, improves moisture resistance of the hair, without compromising the Hair Color or Hair Bleach result.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "10Gew.%" is intended to mean "about 10Gew.%".

Every document cited herein, including any cross referenced or related patent or patent publication, is hereby incorporated herein by reference in its entirety unless expressly excluded or otherwise limited. The citation of any document is not an admission that it is prior art with respect to any document disclosed or claimed herein or that it alone, or in any combination with any other reference or references, teaches, suggests or discloses any such embodiment. Further, to the extent that any meaning or definition of a term in this document conflicts with any meaning or definition of the same term in a document incorporated by reference, the meaning or definition assigned to that term in this document shall govern.

While particular embodiments have been illustrated and described herein, it should be understood that various other changes and modifications may be made without departing from the spirit and scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

## Claims

1. A method for treating hair comprising the steps of:
• applying to the hair a first formulation, comprising an at least bi-functional Brönsted-base which is 4,7,10-Trioxa-1,13-tridecanediamine, 4,9-Dioxa-1,12-dodecanediamine or 1,11-Diamino-3,6,9-trioxaundecane, (1. Step) and
• applying to the hair a second formulation, comprising an at least bi-functional Brönsted-acid which can react with the amine groups of the hair and which is maleic acid, malic acid or a mixture of fumaric acid and itaconic acid (2. Step), and at least one fatty alcohol, which is cetyl alcohol, cetearyl alcohol or 1-dodecanol,
**characterized in that**
• the first formulation is applied before the second formulation is, and the first formulation has a pH of 7 to 12 and the at least bi-functional Bronsted-base is present in a concentration range of 0,1 to 25 percent by weight, and the second formulation has a pH of 1,5 to 7, and the at least bi-functional Bronsted-acid is present in a concentration range of 0,01 to 0,99 percent by weight, and the at least one fatty alcohol, which is cetyl alcohol, cetearyl alcohol or 1-dodecanol, is present in a concentration range of 0,1 to 25 percent by weight.

2. The method of claim 1, **characterized in that** the second formulation also comprises monoethanolamine as an organic base to adjust the pH of the acidic crosslinking formulation to pH 1.5 to 7.

3. The method of any of the preceding claims 1 or 2, **characterized in that** formulation (a) comprises an acidifying agent and formulation (b) comprises an alkalizing agent.

4. The method of any of the preceding claims, **characterized in that** the acidifying agent is an inorganic acid.

5. The method of any of the preceding claims, **characterized in that** the acidifying agent is a mono-functional organic acid.

6. The method of any of the preceding claims, **characterized in that** the acidifying agent is a poly-functional organic acid.

7. The method of any of thr preceding claims, **characterized in that** the alkalizing agent is a an inorganic Bronsted-base.

8. The method of claim 3, **characterized in that** the alkalizing agent is a an organic Bronsted-base.

9. The method of claim 3, **characterized in that** the acidifying agent of the first formulation is the at least bi-functional Bronsted-acid of the second formulation.

10. The method of any of the preceding claims, **characterized in that** the hair is dried inbetween steps (a) and (b) and the drying time is 1 to 60 minutes.

11. The method of claim 10, **characterized in that** a drying device is used for drying the hair.

12. The method of any of the preceding claims, **characterized in that** the formulation of step (a) is left on the hair for 1 to 45 minutes.

13. The method of any of the preceding claims, **characterized in that** the formulation of step (b) is left on the hair for 1 to 45 minutes.

14. The method of any of the preceding claims, **characterized in that** there is a waiting time inbetween the applications of steps (a) and (b) of 1 to 60 minutes.

15. The method of any of the preceding claims, **characterized in that** the formulations of the 1. and of the 2. Steps are independently mixed into a cosmetically acceptable carrier and where the cosmetically acceptable carrier of the formulation of the 1. Step is either identical or not to the cosmetically acceptable carrier of the formulation of the 2. Step .

16. The method of any of the preceding claims, **characterized in that** the formulations of the 1. Step and of the 2. Step are mixed into a commercially available hair coloring or hair bleaching formulation prior to the application to hair.

17. The method of any of the preceding claims, **characterized in that** prior to the application of the 1. Step and of the 2. Step the hair is treated with a thioglycolic acid containing hair care composition.

18. A kit for treating hair comprising two separate formulations (1) and (2), wherein
• formulation (1) comprises an at least bi-functional Bronsted-base of the general formulation X-R-Y, which is 4,7,10-Trioxa-1,13-tridecanediamine, 4,9-Dioxa-1,12-dodecanediamine or 1,11-Diamino-3,6,9-trioxaundecane, and
• formulation (2) comprises an at least bi-functional Bronsted-acid which can react with the amine groups of the hair, which is maleic acid, malic acid or a mixture of fumaric acid and itaconic acid, and at least one fatty alcohol, which is cetyl alcohol, cetearyl alcohol or 1-dodecanol,
**characterized in that**
• formulation (1) has a pH of 7 to 12 and formulation (2) has a pH of 1.5 to 7, and the at least bi-functional Bronsted-base of the first formulation is present in a concentration range of 0.1 percent by weight to 25 percent by weight, and the at least bi-functional Bronsted-acid of the second formulation is present in a concentration range of 0.01 percent by weight to 0.99 percent by weight, and at least one fatty alcohol, which is cetyl alcohol, cetearyl alcohol or 1-dodecanol, is present in a concentration range of 0.1 percent by weight to 25 percent by weight.

19. A kit for treating hair comprising three separate compositions (a) and (b) and (c), wherein
• composition (a) comprises an at least bi-functional Bronsted-base of the general formulation X-R-Y, which is 4,7,10-Trioxa-1,13-tridecanediamine, 4,9-Dioxa-1,12-dodecanediamine or 1,11-Diamino-3,6,9-trioxaundecane, and
• composition (b) comprises an at least bi-functional Bronsted-acid which can react with the amine groups of the hair, which is maleic acid, malic acid or a mixture of fumaric acid and itaconic acid and
• composition (c) comprises at least one fatty alcohol, which is cetyl alcohol, cetearyl alcohol or 1-dodecanol,
**characterized in that**
• formulation (a) has a pH of 7 to 12 and formulation (b) has a pH of 1.5 to 7, and the least bi-functional Bronsted-base of the first formulation is present in a concentration range of 0.1 percent by weight to 25 percent by weight, and the at least bi-functional Bronsted-acid of the second formulation is present in a concentration range of 0.01 percent by weight to 0.99 percent by weight, and the at least one fatty alcohol, which is cetyl alcohol, cetearyl alcohol or 1-dodecanol, is present in a concentration range of 0.1 percent by weight to 25 percent by weight.

20. The kit of any of the preceding claims 18 or 19, **characterized in that** the second formulation also comprises monoethanolamine as an organic base to adjust the pH of the acidic crosslinking formulation to pH 1.5 to 7.

## Patentansprüche

1. Verfahren zur Behandlung von Haar, das folgende Schritte umfasst:
• Auftragen einer ersten Rezeptur auf das Haar, die mindestens eine bifunktionelle Brønsted-Base aufweist, die 4,7,10-Trioxa-1,13-tridecandiamin, 4,9-Dioxa-1,12-dodecandiamin oder 1,11-Diamino-3,6,9-trioxaundecan ist, (1. Schritt) und
• Auftragen einer zweiten Rezeptur auf das Haar, die mindestens eine bifunktionelle Brønsted-Säure, die mit den Amin-Gruppen des Haares reagieren kann und die Maleinsäure, Apfelsäure oder eine Mischung von Fumarsäure und Itaconsäure ist (2. Schritt), und mindestens einen Fettalkohol aufweist, der Cetylalkohol, Cetylstearylalkohol oder 1-Dodecanol ist,
**dadurch gekennzeichnet, dass**
• die erste Rezeptur vor der zweiten Rezeptur aufgetragen wird, die erste Rezeptur einen pH-Wert von 7 bis 12 aufweist, die mindestens bifunktionelle Bronsted-Base in einem Konzentrationsbereich von 0,1 bis 25 Gewichtsprozent vorliegt, die zweite Rezeptur einen pH-Wert von 1,5 bis 7 aufweist, die mindestens bifunktionelle Bronsted-Säure in einem Konzentrationsbereich von 0,01 bis 0,99 Gewichtsprozent vorliegt und der mindestens eine Fettalkohol, der Cetylalkohol, Cetylstearylalkohol oder 1-Dodecanol ist, in einem Konzentrationsbereich von 0,1 bis 25 Gewichtsprozent vorliegt.

2. Verfahren nach Anspruch 1, **dadurch gekennzeichnet, dass** die zweite Rezeptur auch Monoethanolamin als organische Base aufweist, um den pH-Wert der sauren Vernetzungsrezeptur auf einen pH-Wert von 1,5 bis 7 anzupassen.

3. Verfahren nach einem der vorhergehenden Ansprüche 1 oder 2, **dadurch gekennzeichnet, dass** Rezeptur (a) ein Säuerungsmittel aufweist und Rezeptur (b) ein Alkalisierungsmittel aufweist.

4. Verfahren nach einem der vorhergehenden Ansprüche, **dadurch gekennzeichnet, dass** das Säuerungsmittel eine anorganische Säure ist.

5. Verfahren nach einem der vorhergehenden Ansprüche, **dadurch gekennzeichnet, dass** das Säuerungsmittel eine monofunktionelle organische Säure ist.

6. Verfahren nach einem der vorhergehenden Ansprüche, **dadurch gekennzeichnet, dass** das Säuerungsmittel eine polyfunktionelle organische Säure ist.

7. Verfahren nach einem der vorhergehenden Ansprüche, **dadurch gekennzeichnet, dass** das Alkalisierungsmittel eine anorganische Brønsted-Base ist.

8. Verfahren nach Anspruch 3, **dadurch gekennzeichnet, dass** das Alkalisierungsmittel eine organische Brønsted-Base ist.

9. Verfahren nach Anspruch 3, **dadurch gekennzeichnet, dass** das Säuerungsmittel der ersten Rezeptur die mindestens bifunktionelle Brønsted-Säure der zweiten Rezeptur ist.

10. Verfahren nach einem der vorhergehenden Ansprüche, **dadurch gekennzeichnet, dass** das Haar zwischen den Schritten (a) und (b) getrocknet wird und die Trocknungszeit 1 bis 60 Minuten beträgt.

11. Verfahren nach Anspruch 10, **dadurch gekennzeichnet, dass** eine Trocknungseinrichtung zum Trocknen des Haares verwendet wird.

12. Verfahren nach einem der vorhergehenden Ansprüche, **dadurch gekennzeichnet, dass** die Rezeptur des Schrittes (a) für 1 bis 45 Minuten auf dem Haar belassen wird.

13. Verfahren nach einem der vorhergehenden Ansprüche, **dadurch gekennzeichnet, dass** die Rezeptur des Schrittes (b) für 1 bis 45 Minuten auf dem Haar belassen wird.

14. Verfahren nach einem der vorhergehenden Ansprüche, **dadurch gekennzeichnet, dass** zwischen den Auftragungen der Schritte (a) und (b) eine Wartezeit von 1 bis 60 Minuten besteht.

15. Verfahren nach einem der vorhergehenden Ansprüche, **dadurch gekennzeichnet, dass** die Rezepturen des 1. Schritts und des 2. Schritts unabhängig in einen kosmetisch akzeptablen Träger gemischt werden und der kosmetisch akzeptable Träger der Rezeptur des 1. Schritts entweder mit dem kosmetisch akzeptablen Träger der Rezeptur des 2. Schritts identisch ist oder nicht.

16. Verfahren nach einem der vorhergehenden Ansprüche, **dadurch gekennzeichnet, dass** die Rezepturen des 1. Schritts und des 2. Schritts in eine kommerziell verfügbare Haarfärbe- oder Haarbleich-Rezeptur gemischt werden, bevor sie auf das Haar aufgetragen werden.

17. Verfahren nach einem der vorhergehenden Ansprüche, **dadurch gekennzeichnet, dass** vor der Auftragung im 1. Schritt und im 2. Schritt das Haar mit einer Thioglykolsäure-haltigen Haarpflege-Zusammensetzung behandelt wird.

18. Kit zur Haarbehandlung, das zwei separate Rezepturen (1) und (2) umfasst, wobei
• Rezeptur (1) eine mindestens bifunktionelle Bronsted-Base der allgemeinen Formel X-R-Y aufweist, welche 4,7,10-Trioxa-1,13-tridecandiamin, 4,9-Dioxa-1,12-dodecandiamin oder 1,11-Diamino-3,6,9-trioxaundecan ist, und
• Rezeptur (2) eine mindestens bifunktionelle Bronsted-Säure, die mit den Amin-Gruppen des Haares reagieren kann und die Maleinsäure, Apfelsäure oder eine Mischung von Fumarsäure und Itaconsäure ist, und mindestens einen Fettalkohol, der Cetylalkohol, Cetylstearylalkohol oder 1-Dodecanol ist, aufweist,
**dadurch gekennzeichnet, dass**
• Rezeptur (1) einen pH-Wert von 7 bis 12 aufweist, Rezeptur (2) einen pH-Wert von 1,5 bis 7 aufweist, die mindestens bifunktionelle Brransted-Base der ersten Rezeptur in einem Konzentrationsbereich von 0,1 Gewichtsprozent bis 25 Gewichtsprozent vorliegt, die mindestens bifunktionelle Bronsted-Säure der zweiten Rezeptur in einem Konzentrationsbereich von 0,01 Gewichtsprozent bis 0,99 Gewichtsprozent vorliegt und mindestens ein Fettalkohol, der Cetylalkohol, Cetylstearylalkohol oder 1-Dodecanol ist, in einem Konzentrationsbereich von 0,1 Gewichtsprozent bis 25 Gewichtsprozent vorliegt.

19. Kit zur Haarbehandlung, das drei separate Zusammensetzungen (a), (b) und (c) aufweist, wobei
• Zusammensetzung (a) eine mindestens bifunktionelle Bronsted-Base der allgemeinen Formel X-R-Y aufweist, die 4,7,10-Trioxa-1,13-tridecandiamin, 4,9-Dioxa-1,12-dodecandiamin oder 1,11-Diamino-3,6,9-trioxaundecan ist, und
• Zusammensetzung (b) eine mindestens bifunktionelle Bronsted-Säure aufweist, die mit den Amin-Gruppen des Haares reagieren kann und die Maleinsäure, Apfelsäure oder eine Mischung von Fumarsäure und Itaconsäure ist, und
• Zusammensetzung (c) mindestens einen Fettalkohol aufweist, der Cetylalkohol, Cetylstearylalkohol oder 1-Dodecanol ist,
**dadurch gekennzeichnet, dass**
• Rezeptur (a) einen pH-Wert von 7 bis 12 aufweist, Rezeptur (b) einen pH-Wert von 1,5 bis 7 aufweist, die mindestens bifunktionelle Brransted-Base der ersten Rezeptur in einem Konzentrationsbereich von 0,1 Gewichtsprozent bis 25 Gewichtsprozent vorliegt, die mindestens bifunktionelle Bronsted-Säure der zweiten Rezeptur in einem Konzentrationsbereich von 0,01 Gewichtsprozent bis 0,99 Gewichtsprozent vorliegt und der mindestens eine Fettalkohol, der Cetylalkohol, Cetylstearylalkohol oder 1-Dodecanol ist, in einem Konzentrationsbereich von 0,1 Gewichtsprozent bis 25 Gewichtsprozent vorliegt.

20. Kit nach einem der vorhergehenden Ansprüche 18 oder 19, **dadurch gekennzeichnet, dass** die zweite Rezeptur auch Monoethanolamin als organische Base aufweist, um den pH-Wert der sauren Vernetzungsrezeptur auf einen pH-Wert von 1,5 bis 7 anzupassen.

## Revendications

1. Un procédé de traitement des cheveux comprenant les étapes suivantes :
• appliquer sur les cheveux une première préparation, comprenant au moins une base bifonctionnelle selon Brönsted à savoir la 4,7,10-trioxa-1,13- tridécanédiamine, la 4,9-dioxa-1,12-dodécanédiamine ou la 1,11-diamino-3,6,9-trioxaundécane (Étape 1) et
• appliquer sur les cheveux une deuxième préparation, comprenant au moins un acide bifonctionnel selon Brönsted qui peut réagir avec les groupes amines des cheveux à savoir l'acide maléique, l'acide malique ou un mélange d'acide fumarique et d'acide itaconique (Étape 2), et au moins un alcool gras, à savoir l'alcool cétylique, l'alcool cétéarylique ou 1-dodécanol,
**caractérisé en ce que**
• la première préparation est appliquée avant la deuxième préparation, et la première préparation a un pH allant de 7 à 12 et au moins une base bifonctionnelle selon Bronsted se situe dans une plage de concentration allant de 0,1 à 25 pour cent en poids, et la deuxième préparation a un pH allant de 1,5 à 7, et au moins de l'acide bifonctionnel selon Bronsted se situe dans une plage de concentration allant de 0,01 à 0,99 pour cent en poids, et au moins un alcool gras, à savoir l'alcool cétylique, l'alcool cétéarylique ou 1-dodécanol, se situe dans une plage de concentration allant de 0,1 à 25 % en poids.

2. Le procédé selon la revendication 1, **caractérisé en ce que** la deuxième préparation comprend également de la monoéthanolamine comme base organique pour ajuster le pH de la formule de réticulation acide à un pH allant de 1,5 à 7.

3. Le procédé selon l'une quelconque des revendications précédentes 1 ou 2, **caractérisé en ce que** la préparation (a) comprend un agent acidifiant et la préparation (b) comprend un agent alcalinisant.

4. Le procédé selon l'une quelconque des revendications précédentes, **caractérisé en ce que** l'agent acidifiant est un acide inorganique.

5. Le procédé selon l'une quelconque des revendications précédentes, **caractérisé en ce que** l'agent acidifiant est un acide organique monofonctionnel.

6. Le procédé selon l'une quelconque des revendications précédentes, **caractérisé en ce que** l'agent acidifiant est un acide organique polyfonctionnel.

7. Le procédé selon l'une quelconque des revendications précédentes, **caractérisé en ce que** l'agent alcalinisant est une base selon Bronsted inorganique.

8. Le procédé selon la revendication 3, **caractérisé en ce que** l'agent alcalinisant est une base selon Bronsted organique.

9. Le procédé selon la revendication 3, **caractérisé en ce que** l'agent acidifiant de la première préparation est au moins l'acide bifonctionnel selon Bronsted de la deuxième préparation.

10. Le procédé selon l'une quelconque des revendications précédentes, **caractérisé en ce que** les cheveux sont séchés entre les étapes (a) et (b) et le temps de séchage est de 1 à 60 minutes.

11. Le procédé selon la revendication 10, **caractérisé en ce qu'**un dispositif de séchage est utilisé pour le séchage des cheveux.

12. Le procédé selon l'une quelconque des revendications précédentes, **caractérisé en ce que** la préparation de l'étape (a) est laissée sur les cheveux pendant 1 à 45 minutes.

13. Le procédé selon l'une quelconque des revendications précédentes, **caractérisé en ce que** la préparation de l'étape (b) est laissée sur les cheveux pendant 1 à 45 minutes.

14. Le procédé selon l'une quelconque des revendications précédentes, **caractérisé en ce qu'**il y a un temps d'attente entre les applications des étapes (a) et (b) de 1 à 60 minutes.

15. Le procédé selon l'une quelconque des revendications précédentes, **caractérisé en ce que** la préparation des Étapes 1. et 2. sont mélangées indépendamment dans un support cosmétiquement acceptable et dans lequel le support cosmétiquement acceptable de la préparation de l' Étape 1. est identique ou non au support cosmétiquement acceptable de la préparation de l' Étape 2.

16. Le procédé selon l'une quelconque des revendications précédentes, **caractérisé en ce que** les préparations des Étapes 1. et 2. sont mélangées dans une préparation de coloration ou de décoloration des cheveux commercialement disponible avant l'application sur les cheveux.

17. Le procédé selon l'une quelconque des revendications précédentes, **caractérisé en ce qu'**avant l'application de l'Étape 1. et l'Étape 2. les cheveux sont traités avec une composition de soin capillaire contenant un acide thioglycolique.

18. Un kit de traitement capillaire comprenant deux préparations distinctes (1) et (2), dans lequel
• la préparation (1) comprend au moins une base selon Bronsted bifonctionnelle de la préparation générale X-R-Y, à savoir la 4,7,10-trioxa-1,13-tridécanédiamine, la 4,9-dioxa-1,12-dodécanédiamine ou la 1,11-diamino-3,6,9-trioxaundécane, et
• la préparation (2) comprend au moins un acide selon Bronsted bifonctionnel qui peut réagir avec les groupes amines des cheveux, à savoir l'acide maléique, l'acide malique ou un mélange d'acide fumarique et d'acide itaconique, et au moins un alcool gras, à savoir l'alcool cétylique, l'alcool cétéarylique ou 1-dodécanol,
**caractérisé en ce que**
• la préparation (1) a un pH allant de 7 à 12 et la préparation (2) a un pH allant de 1,5 à 7, et au moins la base selon Bronsted bifonctionnelle de la première préparation se situe dans une plage de concentration allant de 0,1 pour cent en poids à 25 pour cent en poids, et au moins un acide bifonctionnel selon Bronsted de la deuxième préparation se situe dans une plage de concentration allant de 0,01 pour cent en poids à 0,99 pour cent en poids, et au moins un alcool gras, à savoir l'alcool cétylique, l'alcool cétéarylique ou 1-dodécanol, se situe dans une plage de concentration allant de 0,1 pour cent en poids à 25 pour cent en poids.

19. Un kit de traitement des cheveux comprenant trois préparations distinctes (a) et (b) et (c), dans lequel
• la composition (a) comprend au moins une base selon Bronsted bifonctionnelle de la préparation générale X-R-Y, qui est la 4,7,10-trioxa-1,13- tridécanédiamine, la 4,9-dioxa-1,12-dodécanédiamine ou la 1,11-diamino-3,6,9-trioxaundécane, et
• la composition (b) comprend au moins un acide selon Bronsted bifonctionnel qui peut réagir avec les groupes amines des cheveux, à savoir l'acide maléique, l'acide malique ou un mélange d'acide fumarique et d'acide itaconique et
• la composition (c) comprend au moins un alcool gras, à savoir l'alcool cétylique, l'alcool cétéarylique ou 1-dodécanol,
**caractérisé en ce que**
• la préparation (a) a un pH allant de 7 à 12 et la préparation (b) a un pH allant de 1,5 à 7, et au moins la base selon Bronsted bifonctionnelle de la première préparation se situe dans une plage de concentration allant de 0,1 pour cent en poids à 25 pour cent en poids, et au moins l'acide selon Bronsted bifonctionnel de la deuxième préparation se situe dans une plage de concentration allant de 0,01 pour cent en poids à 0,99 pour cent en poids, et au moins un alcool gras, à savoir l'alcool cétylique, l'alcool cétéarylique ou le dodécanol-1, se situe dans une plage de concentration allant de 0,1 pour cent en poids à 25 pour cent en poids.

20. Un kit selon l'une quelconque des revendications précédentes 18 ou 19, **caractérisé en ce que** la deuxième préparation comprend également de la monoéthanolamine comme base organique pour ajuster le pH de la préparation de réticulation acide à un pH allant de 1,5 à 7.
